# EUROPÄISCHE PATENTSCHRIFT

(11) **EP 0 629 183 B1**
(45) Veröffentlichungstag und Bekanntmachung des Hinweises auf die Patenterteilung: **20.05.1998**
(21) Anmeldenummer: 93903944.2
(22) Anmeldetag: 10.02.1993
(51) Int. Cl.: C07C 53/126, C07C 59/01, C07C 59/185, C07C 57/03, C07C 57/12, C07C 59/42, C07C 59/44, C07C 51/41

(54) **BASISCHE MAGNESIUM/ZINK-MISCHSEIFEN**
MIXED MAGNESIUM/ZINC BASIC SOAPS
SAVONS MELANGES BASIQUES AU MAGNESIUM/ZINC

(30) Priorität: 19.02.1992 DE 4204886
(43) Veröffentlichungstag der Anmeldung: 21.12.1994
(73) Patentinhaber: NEYNABER CHEMIE GmbH, D-27608 Loxstedt (DE)
(72) Erfinder: WORSCHECH, Kurt, D-2854 Loxstedt (DE); FLEISCHER, Erwin, D-2858 Schiffdorf (DE); LÖFFELHOLZ, Frido, D-2850 Bremerhaven (DE); WEDL, Peter, D-2850 Bremerhaven (DE); JAECKEL, Manfred, D-2854 Loxstedt (DE)
(86) Internationale Anmeldenummer: EP9300321
(87) Internationale Veröffentlichungsnummer: WO9316979

(56) Entgegenhaltungen:
- EP-A- 0 330 097
- FR-A- 2 351 163

## Beschreibung

Die Erfindung liegt auf dem Gebiet der thermoplastischen halogenhaltigen Kunststoffe und betrifft basische Magnesium/Zink-Mischseifen, ein Verfahren zur Herstellung derartiger basischer Magnesium/Zink-Mischseifen und ihre Verwendung als Stabilisatoren in thermoplastischen halogenhaltigen Kunststoffen.

Halogenhaltige Kunststoffe, insbesondere chlorhaltige wie Polyvinylchlorid (PVC), oder daraus hergestellte Formmassen neigen bekanntermaßen unter Hitzebedingungen oder beim Aussetzen an Ultraviolett-Licht zu einem Abbau bzw. zu Zersetzungserscheinungen, die sich beispielsweise in Form von Farbveränderungen äußern. Um dem entgegenzuwirken, werden herkömmlicherweise Stabilisatormischungen zugesetzt, die Blei-, Zinn-, Barium- und/oder Cadmiumverbindungen enthalten. So sind beispielsweise aus der deutschen Offenlegungsschrift DE-A-34 44 259 basische Bleiseifensysteme des Typs 2PbO · Pb (Fettsäurerest)₂ bekannt. Aufgrund physiologischer Überlegungen möchte man die bleihaltigen Stabilisatoren soweit wie möglich ersetzen. Dies gilt insbesondere für halogenhaltige thermoplastische Kunststoffe, die mit Lebensmitteln in Berührung kommen. Als Alternative zu bleihaltigen Stabilisatoren kommen beispielsweise Magnesium- und Zinkverbindungen in Frage. Bei den meisten Magnesium/Zinkstabilisatoren handelt es sich um physikalische Mischungen von Magnesium- und Zinkseifen von höheren Fettsäuren mit 8 bis 22 C-Atomen. Bei diesen Metallseifen bewirkt der Fettsäurerest eine Gleitwirkung und die Metallkomponente die eigentliche Stabilisierung. Um eine hohe Stabilisierung zu erreichen, ist man auf der einen Seite daran interessiert, hohe Gehalte an diesen Seifen in die halogenhaltigen Kunststoffe einzutragen. Mit der Erhöhung des Eintrags an Metallseifen wird aber automatisch auch stets die Menge an eingetragenen Fettsäureresten erhöht, was aber aufgrund der nur bedingten Verträglichkeit der halogenhaltigen Kunststoffe mit den Fettsäureresten problematisch werden kann. So kann es dann bei der Verarbeitung der halogenhaltigen thermoplastischen Kunststoffen zu unerwünschter Belagsbildung an den Werkzeugen wie Extrudierdüsen und dergleichen kommen. Ein noch größeres Problem ist die häufig anzutreffende Überschmierung bei hohen Mengen an eingebrachten Fettsäureresten. Derartige Überschmierungen wirken sich insbesondere bei der Extrusion von halogenhaltigen Kunststoffen oft negativ aus. Desweiteren stauben physikalische Mischungen von Zink- und Calciumseifen bei der Handhabung in unerwünschtem Maße.

In der deutschen Offenlegungsschrift DE-A-38 06 192 wird vorgeschlagen, basische Metallseifen einer Zusammensetzung der Formel (MO)ₙ^{^{·}} M(RCOO)₂, in der MO ein Oxid aus der von CaO, ZnO, MgO, BaO und PbO gebildeten Gruppe, M eines der vorgenannten Metalle und RCOO ein Fettsäurerest bedeuten, als Stabilisator/Gleitmittel-Zusammensetzung für die Kunststoffindustrie zu verwenden. Diese basischen Metallseifen sollen erhalten werden, indem man pulverförmige Fettsäuren mit pulverförmigen Metalloxiden in Gegenwart von Wasser und/oder einer Säure umsetzt. Tatsächlich entstehen bei dieser Festkörperreaktion jedoch Reaktionsmischungen, die nur zu geringem Anteil die basischen Metallseifen enthalten und in noch großen Mengen die Edukte. Damit können die störenden Überschmierungen nicht im gewünschten Ausmaß vermieden werden.

DE-A-26 20 092 betrifft ein Mittel zur Verminderung der Brennbarkeit bzw. der Entflammbarkeit und der Rauchentwicklung von halogenhaltigen Kunststoffen bzw. Kunststoffmassen. Dieses Mittel enthält ein Magnesium-Zink-Komplexsalz, das durch Umsetzung von 2 bis 10 Mol Magnesiumoxid mit 1 Mol eines Zinksalzes einer organischen oder anorganischen Säure hergestellt wurde.

Die Aufgabe der vorliegenden Erfindung bestand darin, bleifreie Stabilisatoren zur Verfügung zu stellen, die bei einem hohen Gehalt an die eigentliche Stabilisierung bewirkenden Metallen einen möglichst niedrigen Gehalt an Fettsäureresten aufweisen. Des weiteren sollten die Stabilisatoren in hohen Ausbeuten erhältlich sein. Zudem sollten die Stabilisatoren in ihrer stabilisierenden Langzeitwirkung nicht bzw. kaum schlechter als Mischungen von Magnesium- und Zinkseifen sein und möglichst in der early-colour-Stabilität besser. Schließlich sollten die Stabilisatoren zumindest weniger stauben als die Ca- und Zinkseifen.

Die Aufgabe wird gelöst durch basische Magnesium/Zink-Mischseifen einer Zusammensetzung der Formel (I), die in einer Schmelzreaktion hergestellt worden sind.

Gegenstand der vorliegenden Erfindung sind demgemäß basische Magnesium/ Zink-Mischseifen einer Zusammensetzung der Formel I

(MgO)ₙZn(OOCR¹)₂ (I)

in der
- R¹: eine geradkettige oder verzweigte Alkyl-, Alkenyl- Hydroxyalkenyloder Hydroxyalkylgruppe mit 7 bis 21 Kohlenstoffatomen oder eine Ketoalkylgruppe mit 11 bis 21 Kohlenstoffatomen und
- n: eine Zahl im Bereich von 0,1 bis 2,5 ist, hergestellt in einer Schmelzreaktion, wobei man einer Zinkseifen der Formel Zn (OOCR¹)₂, mit der bereits angegebenen Bedeutung von R¹, enthaltenden Schmelze Magnesiumoxid oder Magnesiumhydroxid in Mengen von 0,1 bis 2,5 Mol pro Mol Zinkseife zugibt.

Die so hergestellten basischen Magnesium/Zinkseifen unterscheiden sich von denen der deutschen Offenlegungsschrift DE-A-38 06 192. Dies ist qualitativ erkennbar bei Schmelzpunktbestimmungen nach Kofler mittels Kofler'sche Heizbank. So zeigt beispielsweise die erfindungemäße basische Magnesium/Zink-Mischseife (MgO)₂·Zn(stearat)₂ einen scharfen Schmelzpunkt bei 74 °C. Die vergleichbare basische Magnesium/Zinkseife hergestellt nach der DE-A-38 06 192 in der Festkörperreaktion zeigt dagegen einen Schmelzbereich zwischen 90 °C bis 170 °C. Nimmt man eine physikalische Mischung aus 2 mol Magnesiumoxid und 1 mol Zinkstearat findet man einen Schmelzpunkt von 126 °C. Allein diese Vergleiche zeigen schon, daß die erfindungsgemäßen basischen Magnesium/Zink-Mischseifen von denen der deutschen Offenlegungsschrift DE-A-38 06 192 und von reinen physikalischen Mischungen verschieden sind.

Die in der Formel (I) wiedergegebene Gruppe R¹COO- leitet sich ab von gesättigten und/oder ungesättigten Monocarbonsäuren mit 8 bis 22 C-Atomen und/oder von gesättigten oder ungesättigten Hydroxycarbonsäuren mit 8 bis 22 C-Atomen und/oder von Ketofettsäuren mit 12 bis 22 C-Atomen. Die Carbonsäuren und/oder Hydroxycarbonsäuren können natürlicher und/oder synthetischer Herkunft sein. Beispiele für geeignete Monocarbonsäuren sind Laurin-, Myristin-, Palmitin-, Stearin-, Arachin-, Behen-, Laurolein-, Myristolein-, Palmitolein-, Öl- und Erucasäure. Beispiele für geeignete Hydroxymonocarbonsäuren sind Ricinol- und 12-Hydroxystearinsäure. Besonders bevorzugt leitet sich die Gruppe R¹COO- von technischen Mischungen der genannten Fettsäuren ab, wie sie in Form der in der Fettchemie üblichen technischen Gemische nach Druckspaltung von Ölen und Fetten tierischer oder pflanzlicher Herkunft, wie Kokos-, Palmkern-, Sonnenblumen-, Raps-, Rübsen- und Korianderöl und Rindertalg, zugänglich sind. Die Gruppe R¹COO- kann aber auch einen verzweigten Fettsäurerest bedeuten, beispielsweise den Rest der 2-Ethylhexansäure, Isopalmitinsäure oder Isostearinsäure. Ebenso können die Carbonsäurereste R¹COO- sich von Ketofettsäuren mit 12 bis 22 C-Atomen ableiten. Typische und bevorzugte Vertreter dieser Ketofettsäuren sind die verschiedenen Isomeren der Ketostearinsäure, die in Acta Chemica Scandinavica 6, 1157 bis 1174 (1952) beschrieben sind. Von diesen isomeren Ketostearinsäuren sind die 3-, 4-, 5-, 6-, 9 (10)- und 12-Ketostearinsäuren besonders bevorzugt, da diese aus natürlichen Rohstoffen besonders leicht zugänglich sind.

Besonders bevorzugt steht in der Formel (I) die Gruppe R¹COO- für gesättigte Fettsäurereste und/oder Ketofettsäurereste der oben beschriebenen Fettsäuren bzw. Ketofettsäuren.

Gemäß einer vorteilhaften Ausführungsform der Erfindung bedeutet in der Formel (I) n eine Zahl im Bereich von 1 bis 2, das heißt, bevorzugte basische Magnesium/Zink-Mischseifen weisen die Zusammensetzung (MgO)₁₋₂·Zn (OOCR¹)₂ auf. Dabei kann die Zahl natürlich eine ganze oder gebrochene Zahl sein.

Die Erfindung betrifft weiterhin ein Verfahren zur Herstellung von basischen Magenisum/Zink-Mischseifen einer Zusammensetzung der Formel (I), daß dadurch gekennzeichnet ist, daß man Magnesiumoxid oder Magnesiumhydroxid zu einer Zinkseifen der Formel Zn(OOCR¹)₂, mit der bereits angegebenen Bedeutung für R¹, enthaltenden Schmelze in Mengen von 0,1 bis 2,5 Mol pro Mol Zinkseife gibt.

In der Regel werden gleichzeitig mit der Zugabe des Magnesiumoxids oder Magnesiumhydroxids katalytische Mengen an Säuren zugegeben. Die erhaltene Reaktionsmischung wird solange in der Schmelze gehalten, bis das Magnesiumoxid oder Magnesiumhydroxid nahezu vollständig abreagiert hat. Das Ende der Reaktion ist dann erreicht, wenn eine klare oder trübe Schmelze auftritt, die keine mit den Augen noch zu erkennenden Feststoffteilchen mehr enthält. Besonders bevorzugt werden 1 bis 2 Mol Magnesiumoxid oder Magnesiumhydroxid pro Mol Zinkseife zugegeben, besonders bevorzugt wird Magnesiumoxid zugegeben. Des weiteren ist es bevorzugt, in Gegenwart von Säuren in Mengen von 0,001 bis 0,1 Gew.-% - bezogen auf Reaktionsmischung - zu arbeiten. Typische Säuren sind kurzkettige Monocarbonsäuren mit 1 bis 3 Kohlenstoffatomen, wie Essigsäure, sowie Mineralsäuren, deren Anion mit den zu stabilisierenden Kunststoffmassen vertäglich sind, wie Phosphorsäure. Die Temperatur, bei der die Umsetzung zwischen Magnesiumhydroxid oder Magnesiumoxid mit den Zinkseifen erfolgt, liegt über den Schmelztemperaturen und unter den Zersetzungstemperaturen der Zinkseifen. In der Regel liegen die Umsetzungstemperaturen im Bereich von 100 bis 180 °C, je nach dem Schmelzpunkt und Zersetzungspunkt der gewählten Zinkseife. Für Umsetzungen von Zinkstearat liegen die Temperaturen etwa im Bereich von 150°C.

Die in dem Verfahren eingesetzten Zinkseifen können entweder im Handel erstanden werden, oder man kann sie auch in situ selbst erzeugen, indem man Carbonsäuren, Hydroxycarbonsäuren oder Ketofettsäuren der Formel R¹COOH mit Zinksalzen, vorzugsweise mit Zinkoxid, in einem molaren Verhältnis von etwa 2:1 umsetzt. Diese Umsetzung wird vorzugsweise so durchgeführt, daß zunächst die Carbonsäuren R¹COOH geschmolzen werden und in diese Schmelze die Zinksalze, insbesondere das Zinkoxid, zugegeben werden. Die Umsetzung wird solange geführt, bis sich die Fettsäuren praktisch vollständig umgesetzt haben. Die praktisch vollständige Umsetzung kann man erkennen durch Ermittlung der Säurezahl gemäß DIN 53402. Die Umsetzung kann gleichermaBen in Gegenwart von katalytischen Mengen an Säuren beschleunigt werden, wobei die Säuremenge vorzugsweise im Bereich von 0,001 bis 0,1 Gew.-% - bezogen auf Reaktionsmischung - liegt. Als Säure können ebenfalls die schon beschriebenen kurzkettigen Monocarbonsäuren sowie Mineralsäuren eingesetzt werden.

Bevorzugt im Sinne der vorliegenden Erfindung werden die erfindungsgemäßen basischen Magnesium/Zink-Mischseifen nach einem 2-Stufen-Verfahren hergestellt, wobei in der ersten Stufe die Zinkseifen in situ gebildet werden und in der zweiten Stufe die basischen Magnesium/Zink-Mischseifen. Zweckmäßigerweise geht man dabei so vor, daß man in der ersten Stufe die Carbonsäuren R¹COOH, mit der bereits angegebenen Bedeutung für R¹, zusammen mit katalytischen Mengen an Säuren vorlegt und schmilzt. In diese Schmelze werden Zinksalze, bevorzugt Zinkoxid, in solchen stöchiometrischen Mengen zugegeben, daß sich die Zinkseifen Zn(R¹COO)₂ bilden. Im zweiten Reaktionsschritt wird zu den geschmolzenen Zinkseifen Zn(R¹COO)₂ Magnesiumoxid bzw. Magnesiumhydroxid in den bereits angegebenen Mengen sowie erneut die katalytischen Säuremengen zugegeben. Sofern Magnesiumhydroxid zugegeben wird, wird das bei der Umsetzung freiwerdende Reaktionswasser aus der Reaktionsmischung laufend entfernt.

Ein weiterer Gegenstand der vorliegenden Erfindung betrifft die Verwendung von basischen Magnesium/Zink-Mischseifen einer Zusammensetzung der Formel (I), hergestellt nach dem erfindungsgemäßen Verfahren, als Stabilisatoren für thermoplastische halogenhaltige Kunststoffe. Vorzugsweise werden die basischen Magnesium/Zink-Mischseifen in Mengen von 0,05 bis 5, insbesondere 0,2 bis 1 Gewichtsteile auf 100 Gewichtsteile halogenhaltige Kunststoffe verwendet.

Als halogenhaltige Kunststoffe kommen in erster Linie chlorhaltige Homound Copolymere von Vinylchlorid in Betracht. Als Copolymere werden bevorzugt Polymerisate von mindestens 50 Gew.% Vinylchlorid und weiteren polymerisierbaren Monomeren wie Vinylester, Methacrylsäureester, Fumarsäureester, Butadien und Vinylidenchlorid. Besonders bevorzugt werden jedoch Polyvinylchloridhomopolymerisate mit K-Werten von 65 bis 70, die durch Emulsions-, Suspensions- und Massepolymerisation hergestellt werden können. Selbstverständlich kann das PVC auch mit Schlagzähigkeitsverbesserern oder Flow-improvern, insbesondere auf Acrylat-Basis, modifiziert sein.

Die erfindungsgemäßen basischen Magnesium/Zink-Mischseifen werden den halogenhaltigen Kunststoffen auf übliche Weise zugegeben, d. h. vor Verarbeitung der halogenhaltigen Kunststoffe.

Die erfindungsgemäßen basischen Magnesium/Zink-Mischseifen können alleine oder zusammen mit anderen aus dem Stand der Technik bekannten Stabilisatoren verwendet werden. Andere bekannte Stabilisatoren sind Hydrotalcite, die unter dem Handelsnamen Alcamizer^{R} von der Kyowa Chemical Int. vertrieben werden. Diese Handelstypen haben eine Oberfläche nach BET, die unter 30 m²/g liegt. Zusätzlich sind diese Handelstypen in der Regel oberflächenmodifiziert mit Dispergiermitteln wie Natriumstearat. Es können aber auch Hydrotalcite mit größeren Oberflächen oder mit anderen Oberflächenmodifizierungsmitteln verwendet werden, beispielsweise die in den deutschen Offenlegungsschriften DE-A-41 17 034 und DE-A-41 17 035 beschriebenen Hydrotalcite.

Weitere geeignete übliche Stabilisatoren sind synthetische kristalline Natriumsilikate wie die in der deutschen Offenlegungsschrift DE-A-24 12 837 beschriebenen vom Typ NaA und/oder basische Calcium-AluminiumHydroxyPhosphite gemäß der deutschen Patentschrift DE-C-39 41 902.

Vorzugsweise werden die erfindungsgemäßen basischen Magnesium/Zink-Mischseifen zusammen mit Hydrotalciten in beliebigen Verhältnissen eingesetzt. Bevorzugt liegt die eingesetzte Menge an Hydrotalcit im Bereich von 0,1 bis 3, insbesondere 0,2 bis 1 Gewichtsteilen auf 100 Gewichtsteile halogenhaltige Kunststoffe.

Die erfindungsgemäßen basischen Magnesium/Zink-Mischseifen können auch zusammen mit für thermoplastische halogenhaltige Kunststoffe üblichen Weichmachern, Gleitmitteln, Trennmitteln und/oder Co-Stabilisatoren eingesetzt werden. Bevorzugt werden sie mit einer oder mehreren üblichen Weichmachern, Gleitmitteln, Trennmitteln und/oder Co-Stabilisatoren ausgewählt aus der Gruppe
a) Fettalkohole mit 8 bis 22 Kohlenstoffatomen,
b) Ester von monofunktionellen Alkanolen mit 1 bis 22 Kohlenstoffatomen mit gegebenenfalls hydroxysubstituierten Fettsäuren mit 8 bis 34 Kohlenstoffatomen oder mit Ketofettsäuren mit 16 bis 22 Kohlenstoffatomen,
c) Ester von epoxidierten Fettsäuren mit 16 bis 22 Kohlenstoffatomen mit monofunktionellen Alkanolen mit 1 bis 8 Kohlenstoffatomen,
d) Vollester von Dicarbonsäuren und Polycarbonsäurne mit 3 oder 4 Carboxylgruppen und mit 3 bis 24 Kohlenstoffatomen mit monofunktionellen Alkanolen mit 8 bis 22 Kohlenstoffatomen,
e) Partial- und Vollester von Polyolen mit 2 bis 15 Kohlenstoffatomen und 2 bis 6 Hydroxylgruppen mit gegebenenfalls hydroxysubstituierten Fettsäuren mit 8 bis 34 Kohlenstoffatomen oder Ketofettsäuren mit 16 bis 22 Kohlenstoffatomen,
f) Vollester von Polyolen mit 2 bis 15 Kohlenstoffatomen und 2 bis 6 Hydroxylgruppen mit epoxidierten, ungesättigten Fettsäuren mit 16 bis 22 Kohlenstoffatomen,
g) Fettketone der Formel (II)

   R²-CO-R³ (II)

   in der R² und R³, die gleich oder verschieden sein können und Alkylgruppen mit 5 bis 21 Kohlenstoffatomen bedeuten,
h) β-Diketone der Formel (III)

   R⁴-CO-CH₂-CO-R⁵ (III)

   in der R⁴ eine Alkylgruppe mit 8 bis 22 Kohlenstoffatomen und R⁵ eine Benzoylgruppe bedeuten,
i) Diamide von Fettsäuren mit 8 bis 22 Kohlenstoffatomen mit Alkylendiaminen mit 2 bis 6 Kohlenstoffatomen,
k) Kohlenwasserstoffwachsen wie Polyethylenwachse und Paraffinen,
l) oxidierte Polyethylenwachse mit einem Zahlenmittel der Molmassen im Bereich von 3000 bis 9000,
m) hydroxylgruppenhaltigen Isocyanurate und
n) sekundäre und/oder tertiäre Ester der phosphorigen Säure wie Di-alkylarylphosphite, Alkyldiarylphosphite, deren Alkylgruppe 2 bis 22 C-Atome aufweisen und deren Arylgruppe eine Phenylgruppe ist, eingesetzt.

Bei der obigen Aufzählung wurde nicht zwischen Weichmacher, Trennmittel, Gleitmittel und Co-Stabilisatoren unterschieden, da die Verbindungen mehrere Eigenschaften in sich vereinigen können.

Die aufgezählten Verbindungen sind an sich bekannte Verbindungen, die im Handel angeboten werden und die in üblichen Mengen zugegeben werden können.

Von den für thermoplastische Kunststoffe üblichen Weichmachern, Gleitmitteln, Trennmitteln und/oder Co-Stabilisatoren, vorzugsweise ausgewählt aus den Gruppen a) bis n), werden besonders solche bevorzugt, die bei Raumtemperatur fest sind bzw. in den eingesetzten Mengen zusammen mit den basischen Magnesium/Zink-Mischseifen ein bei Raumtemperatur festes Prdoukt ergeben.

Die für die thermoplastischen Kunststoffe üblichen Weichmacher, Gleitmittel, Trennmittel und/oder Co-Stabilisatoren können auf bekannte Weise mit den basischen Magnesium/Zink-Mischseifen vermischt werden.

Es ist aber auch möglich, die erfindungsgemäßen basischen Magnesium/Zink-Mischseifen in den üblichen Gleitmitteln zu lösen oder zu suspendieren. Übliche Gleitmittel sind beispielsweise Fettalkohole (siehe Gruppe a). Ester von monofunktionellen Alkanolen und Fettsäuren (siehe Gruppe b), Dicarbonsäureester (Gruppe d), Polyolpartial- und Vollester von Fettsäuren (Gruppe e), Fettketone (Gruppe g), Diamide (Gruppe i), Kohlenwasserstoffwachse (Gruppe k) und oxidierte Polyethylenwachse (Gruppe l). Besonders bevorzugte Gleitmittel sind Glycerin- oder Pentaerythritpartialester von Fettsäuren mit 16 bis 22 C-Atomen. Sofern man die basischen Magnesium/Zink-Mischseifen in den üblichen Gleitmitteln lösen oder suspendieren möchte, erreicht man dies durch ein in-situ-Verfahren. Danach wird zu der Schmelze der Zinkseifen die Gleitmittel gegeben und aufgeschmolzen, bevor durch Zugabe von Magnesiumoxid bzw. -hydroxid die erfindungsgemäßen basischen Magnesium/Zink-Mischseifen hergestellt werden.

Diese Verfahrensweise empfiehlt sich dann, wenn die Zinkseifen der Schmelze besonders hochviskos sind und man durch Zugabe der Gleitmittel eine niedrigviskosere Schmelze erreichen kann, da niedrigviskose Schmelzen schneller und vollständiger mit dem Magnesiumoxid bzw. -hydroxid abreagieren können.

Bei Verwendung der erfindungsgemäßen Magnesium/Zink-Mischseifen zeigen PVC-Massen eine mit Mischungen von Magnesium- und Zinkseifen vergleichbaren Stabilitätsabbruch. Besser als bei üblichen Mischungen von Magnesium- und Zinkseifen ist die early-color-Stabilität bei PVC mit den erfindungsgemäßen basischen Magnesium/Zink-Mischseifen, d. h. die Zeit, bei der erste leichte Verfärbungen aber noch keine starken Verfärbungen auftreten. Je länger die early-color-Stabilität ist, desto häufiger kann man PVC verarbeiten ohne große Farbveränderungen des PVC's befürchten zu müssen. Als besonderer Vorteil ist festzuhalten, daß insbesondere PVC-Massen trotz des für die Stabilisierung erforderlichen Gehalts an Magnesium und Zink keine Überschmierungen aufweisen. Dies ist insbesondere im Extruder zu sehen, wo der Druckaufbau geringer abfällt als für Mischungen von Magnesiumoxid und Zinkseifen oder von Magnesiumseifen und Zinkseifen.

Außerdem sind die basischen Magnesium/Zink-Mischseifen nicht-staubende Produkte.

### Beispiele

### A) Herstellung von basischen Magnesium/Zink-Mischseifen

### Beispiel 1

### Zn-laurat·MgO

Ein Vierhalsrundkolben mit Rührer, Thermometer und absteigendem Kühler wurde mit 158,9 g (0,80 Mol) Laurinsäure und 0,05 g 99 %-iger Essigsäure als Katalysator beschickt. In die geschmolzene Fettsäure wurde bei 140 bis 150 °C portionsweise 32,3 g (0,40 Mol) Zinkoxid eingetragen und Vakuum angelegt. Dieses wurde kontinuierlich verstärkt, bis ein Vakuum von 30 hPa erreicht war. Insgesamt wurde 1 1/2 h unter Vakuum gerührt. Der Reaktionskolben wurde belüftet und 15,9 g (0,40 Mol) Magnesiumoxid sowie die gleiche Menge an Essigsäure wie zu Beginn als Katalysator hinzugegeben. Das Vakuum wurde erneut angelegt und innerhalb 1 h auf 20-30 hPa kontinuierlich verstärkt. Insgesamt wurde in der 2. Reaktionsstufe 2 1/2 h gerührt. Abschließend lag eine bräunliche, milchig-getrübte, schwach viskose Schmelze ohne Feststoffanteile vor. Das Schmelzprodukt wurde zum Erstarren in eine Wanne überführt. Erhalten wurde eine gelblich-braunstichige, wachsige Masse mit einem Schmelzpunkt von 105 °C.

### Beispiel 2

### Zn-behenat·MgO

Analog Beispiel 1 wurden 173,5 g (0,5 Mol) technische Behensäure (mit einer Säurezahl gemäß DIN 53402 SZ 165) und 0,05 g Essigsäure vorgelegt. Die Umsetzung zur Zinkseife in der 1. Stufe wurde, wie im Beispiel 1 beschrieben, nach Zugabe von 20,8 g (0,25 Mol) ZnO durchgeführt. Nach der abgeschlossenen Neutralseifenbildung wurde die gleiche Menge an Essigsäure wie zu Beginn sowie 10,3 g (0,25 Mol) MgO eingetragen. Unter den gleichen Bedingungen wie bei Beispiel 1 beschrieben erfolgte die Umsetzung. Hiernach enthielt das Schmelzprodukt keine ungelösten Anteile mehr. Nach dem Erstarren des Produkts wurde eine braunstichige, wachsige Masse mit einem Schmelzpunkt von 118 °C erhalten.

### Beispiel 3

### Zn-12-ketostearat·MgO

Analog Beispiel 1 wurden 170,5 (0,56 Mol) technische 12-Ketostearinsäure (SZ = 187) und 0,05 g Essigsäure vorgelegt. Die Reaktion zur neutralen Zinkseife wurde wie im Beispiel 1 beschrieben nach Zugabe von 23,1 g (0,28 Mol) ZnO durchgeführt. Nach der Seifenbildung wurden die gleiche Menge an Essigsäure wie zu Beginn sowie 11,5 g (0,28 Mol) MgO eingetragen. Nach der Umsetzung lag eine braunstichige, schwach getrübte, schwach viskose Schmelze ohne Bodensatz vor, die nach dem Abkühlen eine braunstichige, wachsige Masse mit einem Schmelzpunkt von 126 °C ergab.

### Beispiel 4

### Zn-stearat·2 MgO

Analog Beispiel 1 wurden 158,0 g (0,58 Mol) technische Stearinsäure (SZ = 207) und 0,05 g Essigsäure vorgelegt. Die Umsetzung zur Zinkseife in der 1. Stufe wurde, wie im Beispiel 1 beschrieben, nach Zugabe von 23,8 g (0,29 Mol) ZnO durchgeführt. Nach der abgeschlossenen Neturalseifenbildung wurde die gleiche Menge an Essigsäure wie zu Beginn sowie 23,4 g (0,58 Mol) MgO eingetragen. Unter den gleichen Bedingungen wie bei Beispiel 1 beschrieben, erfolgte die Umsetzung. Hiernach enthielt das Schmelzprodukt keine ungelösten Anteile mehr. Es lag eine gelbliche, schwach dünnsämige Schmelze vor, die beim Abkühlen zu einer schwach gelblichen, sprödwachsigen Masse mit einem Schmelzpunkt von 74 °C erstarrte.

### Beispiel 5

### Zn-12-hydroxystearat·MgO

Analog Beispiel 1 wurden 214,5 g (0,68 Mol) technische 12-Hydroxystearinsäure (SZ = 180) und 0,05 g Essigsäure vorgelegt. Zur Bildung der Zinkseife wurden 28,0 g (0,34 Mol) ZnO verwendet. Dann wurde die gleiche Menge Essigsäure wie zu Beginn sowie 13,7 g (0,34 Mol) MgO eingetragen. Es wurde weiter wie im Beispiel 1 verfahren. Das Reaktionsprodukt lag ohne Bodenkörper als bräunliche, sämige Schmelze vor, die beim Abkühlen erstarrte. Es wurde ein braunstichig-gelbliches, wachsiges Produkt mit einem Schmelzpunkt von 95 °C erhalten.

### Beispiel 6

### Zn-isostearat·MgO

Analog Beispiel 1 wurden 170,2 g (0,57 Mol) technische Isostearinsäure (SZ ungefähr 190) und 0,05 g Essigsäure vorgelegt. Die Umsetzung zur Zinkseife in der 1. Stufe wurde nach Zugabe von 23,4 g (0,285 Mol) ZnO durchgeführt. Dann wurde die gleiche Menge an Essigsäure wie zu Beginn sowie 11,5 g (0,285 Mol) MgO eingetragen. Es wurde weiter wie im Beispiel 1 verfahren. Es lag abschließend eine karamellfarbene, dünnsämige Schmelze ohne Bodenkörper vor, die nach dem Erstarren eine bräunliche, plastillinartige Masse ergab.

### B) Anwendungstests

100 Gewichtsteile Suspension-PVC mit einem K-Wert von 68 (Corvic S 68/173^{R}) wurden vermischt mit den in Tabelle 1 aufgeführten Verbindungen in den dort aufgeführten Mengen (Mengenangabe in Gewichtsteile; phr). In dem erfindungsgemäßen Beispiel B1) wurde Zinkstearat·2MgO (Formel (MgO)₂Zn(stearat)₂) gemäß Beispiel 4 getestet. Als Vergleich wurde eine Mischung von Magnesiumstearat und Zinkstearat sowie Magnesiumoxid und Zinkstearat gewählt. Die Menge an Vergleichsmischung wurde so gewählt, daß der Magnesium- und Zinkgehalt dem des Beispiels 4 entspricht.

**Tabelle 1:**

| **PVC-Zusammensetzung** | | | |
|---|---|---|---|
| Produkte | B1 | Vgl. 1 | Vgl. 2 |
| PVC | 100 | 100 | 100 |
| Schlagzähigkeitsverbesserer u. Flow-modifier auf Acrylatbasis (Paraloid^{R}) | 9 | 9 | 9 |
| Titandioxid (Kronos 2220^{R}) | 4 | 4 | 4 |
| Kreide (Omyalite 95 T^{R}) | 4 | 4 | 4 |
| Hydrotalcit (Alcamizer 4^{R}) | 0,6 | 0,6 | 0,6 |
| Alkylarylphosphit (Stabiol VP 1929^{R}) | 0,3 | 0,3 | 0,3 |
| Trishydroxyethylcyanurat | 0,3 | 0,3 | 0,3 |
| Benzoylstearylmethan | 0,1 | 0,1 | 0,1 |
| Glycerindistearat | 1 | 1 | 1 |
| Cetylstearylpalmitatstearat | 0,5 | 0,5 | 0,5 |
| Beispiel 4 | 0,5 | - | - |
| Zinkstearat | - | 0,45 | 0,45 |
| Magnesiumstearat | - | 0,8 | - |
| Magnesiumoxid | - | - | 0,06 |

Die in Tabelle I wiedergegebene Zusammensetzung wurde zu einer Formmasse verarbeitet. Bei der Verarbeitung der Formmasse wurde ein handelsüblicher Doppelschneckenextruder (Reifenhäuser Doppelschneckenextruder BT 55/16) verwendet. Bei der Herstellung von Vierkantrohren (63x3 mm) wurden folgende Parameter gewählt:
Zylindertemperatur: 170 / 170 / 170 / 150 / 150 °C
Kopftemperatur: 155 / 170 / 170 °C
Schnecke: K 6/2
Schneckendrehzahl: 25 Upm.

Während der Verarbeitung wurde der Massedruck 2 (in bar) bestimmt, der sich vor dem formgebenden Werkzeug aufbaut. Des weiteren wurde die Maschinenauslastung (in %) abgelesen. Die Ergebnisse sind in Tabelle 2 zusammengestellt.

**Tabelle 2:**

| Parameter | B1) | Vgl. 1 | Vgl. 2 |
|---|---|---|---|
| Massedruck 2 | 311 | 245 | 289 |
| Auslastung | 42 | 39 | 39 |

Tabelle 2 zeigt, daß der Massedruck 2 vor dem formgebenden Werkzeug bei dem erfindungsgemäßen Beispiel deutlich höher ist als bei den Vergleichsbeispielen, d. h. die Wahrscheinlichkeit zur Überschmierung ist deutlich geringer.

Die hergestellten Vierkantrohre wurden auf ihre Reservestabilität geprüft. Hierzu wurden 1 x 1 cm große Probekörper ausgeschnitten und in einem Trockenschrank (Heraeus) mit rotierenden Horden gegeben, der eine Temperatur von 180 °C aufwies.

In Abständen von 15 Minuten wurden Proben entnommen. Bestimmt wurde die Zeit, nach der eine erste leichte Gelbfärbung und eine deutliche Verfärbung auftrat (= early-colour-Stabilität). Die Ergebnisse sind in Tabelle 3 zusammengefaßt.

**Tabelle 3**

| **Reservestabilität** | | | |
|---|---|---|---|
| Beispiel | early-color | | Stabilitätsabbruch in Minuten |
| | erste | deutliche | |
| | Verfärbung in Minuten | | |
| 1 | 30 | 60 | 150 |
| Vgl. 1 | 15 | 30 | 150 |
| Vgl. 2 | 30 | 60 | 150 |

Aus Tabelle 3 wird deutlich, daß die erfindungsgemäßen basischen Magnesium/Zink-Mischseifen eine bessere early-color-Stabilität haben als reine physikalische Mischungen der Magnesium- und Zinkseifen (Vgl. 1).

Des weiteren wurde die dynamische Stabilität geprüft. Dazu wurde ein Plastigraph (Brabender-Plasticorder PLE 330) eingesetzt. Die Kammertemperatur betrug 200 °C, die Kneterdrehzahl 30 min⁻¹.

Die Untersuchungen wurden an Walzfellen durchgeführt, die aus 30 g der in Tabelle 1 angegebenen Zusammensetzungen B1) und Vgl. 1 auf einem Laborwalzwerk der Abmessung 450x220 mm (Firma Berstorff) bei einer Walzentemperatur von 170 °C und einer Walzendrehzahl von 12,5 Upm im Gleichlauf im Verlauf von 5 Minuten hergestellt worden sind. Die Walzfelle wurden anschließend in den Plastigraphen gegeben. Es wurde bestimmt nach wieviel Minuten eine leichte Verfärbung auftrat (early-color-Stabilität) und eine Dunkelbraunverfärbung (Stabilitätsabbruch). In Tabelle 3 sind die Zeitangaben zur Stabilität zu finden.

**Tabelle 4:**

| **Dynamische Stabilität** | | |
|---|---|---|
| Beispiel | early-color-Stabilität leichte Verfärbung (in Minuten) | Stabilitätsabbruch (in Minuten) |
| 1 | 22 | 37 |
| Vgl. 1 | 7 | 37 |

Tabelle 4 bestätigt die Ergebnisse der Tabelle 3, wonach die PVC-Masse mit dem erfindungsgemäßen Beispiel erst später Verfärbungen zeigt als die Mischung von Magnesium- und Zinkseifen.

## Patentansprüche

1. Basische Magnesium/Zink-Mischseifen einer Zusammensetzung der Formel I
(MgO)ₙZn(OOCR¹)₂ (I)
in der
R¹ eine geradkettige oder verzweigte Alkyl-, Alkenyl-, Hydroxyalkenyl-oder Hydroxyalkylgruppe mit 7 bis 21 Kohlenstoffatomen oder eine Ketoalkylgruppe mit 11 bis 21 Kohlenstoffatomen und
n eine Zahl im Bereich von 0,1 bis 2,5 ist,
hergestellt in einer Schmelzreaktion, wobei man einer Zinkseifen der Formel Zn (OOCR¹)₂, mit der bereits angegebenen Bedeutung von R¹, enthaltenden Schmelze Magnesiumoxid oder Magnesiumhydroxid in Mengen von 0,1 bis 2,5 Mol pro Mol Zinkseife zugibt.

2. Basische Magnesium/Zink-Mischseifen nach Anspruch 1, dadurch gekennzeichnet, daß in der Formel (I) n eine Zahl im Bereich von 1 bis 2 ist.

3. Verfahren zur Herstellung von basischen Magnesium/Zink-Mischseifen einer Zusammensetzung der Formel (I)
(MgO)ₙZn(OOCR¹)₂ (I)
in der R¹ eine geradkettige oder verzweigte Alkyl-, Alkenyl-, Hydroxyalkenyl- oder Hydroxyalkylgruppe mit 7 bis 21 Kohlenstoffatomen oder eine Ketoalkylgruppe mit 11 bis 21 Kohlenstoffatomen und
n eine Zahl im Bereich von 0,1 bis 2,5 ist, dadurch gekennzeichnet, daß man Magnesiumoxid oder Magnesiumhydroxid zu einer Schmelze, die die Zinkseifen der Formel Zn(OOCR¹)₂,
mit der angegebenen Bedeutung von R¹ enthält, in einer Menge von 0,1 bis 2,5 Mol Magnesiumoxid oder Magnesiumhydroxid pro Mol Zinkseife zugibt.

4. Verfahren nach Anspruch 3, dadurch gekennzeichnet, daß man 1 bis 2 Mol Magnesiumoxid oder Magnesiumhydroxid pro Mol Zinkseife zugibt.

5. Verfahren nach Anspruch 3 oder 4, dadurch gekennzeichnet, daß man Magnesiumoxid zugibt.

6. Verfahren nach einem der Ansprüche 3 bis 5, dadurch gekennzeichnet, daß man das Reaktionsgemisch auf Temperaturen im Bereich von 100 bis 180 °C erhitzt.

7. Verfahren nach einem der Ansprüche 3 bis 6, dadurch gekennzeichnet, daß zusätzlich Säuren in einer Menge von 0,001 bis 0,1 Gew.-%, bezogen auf Reaktionsmischung, zugegeben sind.

8. Verwendung von basischen Magnsium/Zink-Mischseifen einer Zusammensetzung der Formel (I) nach Anspruch 1 oder 3 als Stabilisatoren für thermoplastische halogenhaltige Kunststoffe.

## Claims

1. Basic magnesium/zinc mixed soaps with a composition corresponding to formula I:
(MgO)ₙZn(OOCR¹)₂ (I)
in which
R¹ is a linear or branched alkyl, alkenyl, hydroxyalkenyl or hydroxyalkyl group containing 7 to 21 carbon atoms or a ketoalkyl group containing 11 to 21 carbon atoms and
n is a number of 0.1 to 2.5,
produced in a melt-phase reaction, magnesium or magnesium hydroxide being added to a melt containing zinc soaps corresponding to the formula Zn (OOCR¹)₂, where R¹ is as already defined, in quantities of 0.1 to 2.5 moles per mole of zinc soap.

2. Basic magnesium/zinc mixed soaps as claimed in claim 1, characterized in that n in formula (I) is a number of 1 to 2.

3. A process for the production of basic magnesium/zinc mixed soaps with a composition corresponding to formula I:
(MgO)ₙZn(OOCR¹)₂ (I)
in which R¹ is a linear or branched alkyl, alkenyl, hydroxyalkenyl or hydroxyalkyl group containing 7 to 21 carbon atoms or a ketoalkyl group containing 11 to 21 carbon atoms and n is a number of 0.1 to 2.5, characterized in that magnesium oxide or magnesium hydroxide is added to a melt containing zinc soaps corresponding to the formula Zn (OOCR¹)₂, where R¹ is as already defined, in quantities of 0.1 to 2.5 moles per mole of zinc soap.

4. A process as claimed in claim 3, characterized in that 1 to 2 moles of magnesium oxide or magnesium hydroxide are added per mole of zinc soap.

5. A process as claimed in claim 3 or 4, characterized in that magnesium oxide is added.

6. A process as claimed in any of claims 3 to 5, characterized in that the reaction mixture is heated to temperatures of 100 to 180°C.

7. A process as claimed in any of claims 3 to 6, characterized in that acids are additionally added in a quantity of 0.001 to 0.1 % by weight, based on the reaction mixture.

8. The use of the basic magnesium/zinc mixed soaps with a composition corresponding to formula (I) claimed in claim 1 or 3 as stabilizers for halogen-containing thermoplastics.

## Revendications

1. Savons mixtes magnésium/zinc basiques d'une composition de la formule I,
(MgO)ₙZn(OOCR¹)₂ (I)
dans laquelle,
R¹ est un radical alkyle, alkényle, hydroxyalkényle ou hydroxyalkyle à chaîne droite ou ramifié, ayant de 7 à 21 atomes de carbone ou un groupe cétoalkyle ayant de 11 à 21 atomes de carbone,
et n est un nombre dans la zone de 0,1 à 2,5,
fabriqués dans une réaction par fusion, dans laquelle on ajoute à un produit de fusion contenant le savon de zinc de formule Zn(OOCR¹)₂, ayant la signification déjà mentionnée de R¹, de l'oxyde de magnésium ou de l'hydroxyde de magnésium en quantités allant de 0,1 à 2,5 mol par mole de savon de zinc.

2. Savons mixtes magnésium/zinc selon la revendication 1, caractérisés en ce que dans la formule (I) n est un nombre dans la zone de 1 à 2.

3. Procédé de préparation de savons mixtes magnésium/zinc basiques d'une composition de formule I,
(MgO)ₙZn(OOCR¹)₂ (I)
dans laquelle,
R¹ est un radical alkyle, alkényle, hydroxyalkényle ou hydroxyalkyle, à chaîne droite ou ramifié, ayant de 7 à 21 atomes de carbone ou un groupe cétoalkyle ayant de 11 à 21 atomes de carbone,
et n est un nombre dans la zone de 0,1 à 2,5 caractérisé en ce qu'
on ajoute de l'oxyde de magnésium ou de l'hydroxyde de magnésium à un produit de fusion qui renferme les savons zinciques de formule,
Zn(OOCR¹)₂
avec la signification indiquée de R¹, en une quantité allant de 0,1 à 2,5 mol d'oxyde de magnésium ou d'hydroxyde de magnésium par mol de savon zincique.

4. Procédé selon la revendication 3,
caractérisé en ce qu'
on ajoute 1 à 2 mol d'oxyde de magnésium ou d'hydroxyde de magnésium par mol de savon zincique.

5. Procédé selon la revendication 3 ou 4,
caractérisé en ce qu'
on ajoute de l'oxyde de magnésium.

6. Procédé selon l'une des revendications 3 à 5,
caractérisé en ce qu'
on chauffe le mélange réactionnel à des températures dans la plage de 100 à 180°C.

7. Procédé selon l'une des revendications 3 à 6,
caractérisé en ce que
des acides en supplément sont ajoutés en une quantité allant de 0,001 à 0,1 % en poids, rapporté au mélange réactionnel.

8. Utilisation des savons mixtes magnésium/zinc basiques d'une composition de la formule (I) selon la revendication 1 ou la revendication 3, comme agents stabilisants pour des matières plastiques contenant des halogènes thermoplastiques.
